# EUROPÄISCHE PATENTSCHRIFT

(11) **EP 0 940 181 B1**
(45) Veröffentlichungstag und Bekanntmachung des Hinweises auf die Patenterteilung: **14.04.2004**
(21) Anmeldenummer: 99103154.3
(22) Anmeldetag: 18.02.1999
(51) Int. Cl.: B01L 3/00, G01N 33/48, G01N 33/50, G01N 33/53

(54) **Pinplatte mit aufsteckbarer oder aufklebbarer Halterung**
Pin plate with holder attached to it by plugging or gluing
Plaque à pointes avec un support attaché par brochage ou par adhésion

(30) Priorität: 03.03.1998 DE 29803626 U
(43) Veröffentlichungstag der Anmeldung: 08.09.1999
(73) Patentinhaber: Pache, Wolfgang, 79294 Sölden (DE)
(72) Erfinder: Pache, Wolfgang, 79294 Sölden (DE)
(74) Vertreter: Zimmermann, Günter, Dr.

(56) Entgegenhaltungen:
- EP-A- 0 042 755
- EP-A- 0 154 686
- WO-A-96/02836
- DE-C- 19 501 298
- GB-A- 2 300 258
- US-A- 4 276 259

## Beschreibung

Die Erfindung bezieht sich auf eine Pinplatte zur Durchführung von Tests in Flüssigkeiten mit in mindestens eine Reihe angeordneten Vorsprüngen auf der Unterseite, wobei eine Halterung zum Transport der Pinplatte vorgesehen ist.

Pinplatten werden für automatisch oder manuell auswertbare Enzymimmunoassays zur Bestimmung beispielsweise von Hormonen in Körperflüssigkeiten verwendet. Die Pinplatten weisen in einer Reihe oder in mehreren regelmäßigen Reihen angeordnete Vorsprünge auf ihrer Unterseite auf. Diese Vorsprünge werden als Pins bezeichnet und tragen auf ihrer Oberfläche immobilisierte Testreagenzien wie Antikörper oder Antigene. Beim Durchführen der Tests wird die Pinplatte auf eine Gegenplatte gelegt, welche in einer Reihe oder in mehreren regelmäßigen Reihen angeordnete Näpfchen aufweist, deren Öffnungen auf ihrer Oberseite angeordnet sind. Die Vorsprünge der Pinplatte sind dabei so ausgelegt, daß jeweils ein Vorsprung in ein Näpfchen bei übereinanderliegenden Platten paßt. In der Gegenplatte mit den Näpfchen, welche auch als sogenannte Mikrowells bezeichnet werden, befinden sich die zu testenden flüssigen Proben, beispielsweise die Körperflüssigkeiten von Patienten. Beim Übereinanderlegen der Pinplatte und der Gegenplatte kommen die Vorsprünge mit dem immobilisierten festen Reagenz auf ihrer Oberfläche mit den in den Näpfchen befindlichen Flüssigkeiten in Kontakt, wodurch eine Testreaktion in Gang kommt. Durch weitere Reaktionen und Waschschritte wird eine Veränderung, wie beispielsweise eine Farbreaktion, in der zu untersuchenden Flüssigkeit hervorgerufen. Für die Auswertung werden dann die Platten in ein Auswertgerät, beispielsweise ein Spektrometer, geschoben, das die Proben einzeln vermißt und die Ergebnisse aufzeichnet. Die Veränderung in der zu untersuchenden Flüssigkeit kann eine Farbänderung sein. Die Pinplatte und die Gegenplatte sind üblicherweise durchsichtig ausgebildet. Mit dem Spektrometer wird die optische Eigenschaft der angefärbten Flüssigkeit, beispielsweise die Lichttransmission bei bestimmten Wellenlängen, für jedes einzelne Näpfchen gemessen, so daß die Konzentration und die Art der in der Flüssigkeit befindlichen Stoffe bestimmt werden kann.

Eine Pinplatte ist durch das Gebrauchsmuster 9015317 bekannt. Diese bekannte Pinplatte weist Schwächungslinien auf, so daß ein mechanisches Teilen der Pinplatte entlang dieser Schwächungslinien möglich ist. Somit können beim Durchführen der Tests auch kleinere Pinplatten verwendet werden, welche beispielsweise nur aus einer Pinreihe bestehen. Das Auflegen auch der kleinen Pinplatten auf die Gegenplatten erfolgt üblicherweise mit der Hand, indem die Pinplatten an der seitlichen Umgrenzung angefaßt werden. Hierbei kann es nachteiligerweise zu einem Kontakt mit den Pins oder der zu untersuchenden Flüssigkeit kommen, welcher gerade bei medizinischen Tests unbedingt zu vermeiden ist. Insbesondere kann ein Kontakt beim Waschen der Pinplatte in der Waschflüssigkeit mit den Fingern auftreten.

Eine Pinplatte der eingangs genannten Art ist durch die EP 0 154 686 A2 bekannt. Bei der Halterung handelt es sich um einen Bügel, welcher an zwei sich gegenüberliegenden Seiten der Pinplatte angebracht ist. Der Bügel ist nachteiligerweise umständlich zu befestigen. Bei mehreren nebeneinander angeordneten Pinplatten, wie es bei der Durchführung von Tests üblich ist, wirken die seitlich über die Pinplatten hinausragenden Bügel störend.

Die Aufgabe der Erfindung besteht somit darin, eine Pinplatte der eingangs genannten Art so weiterzuentwickeln, daß eine einfache und schnelle Handhabung der Pinplatte bei geringem seitlichem Platzbedarf gewährleistet ist, wobei ein Kontakt mit den Pins, eine Verunreinigung der Platte bei der Durchführung der Tests und eine Kontamination des Anwenders durch Körperflüssigkeiten oder Waschflüssigkeiten vermieden werden soll.

Zur Lösung dieser Aufgabe wird erfindungsgemäß vorgeschlagen, daß die Halterung gemäß dem Oberbegriff des Anspruchs 1 entsprechend dem kennzeichnenden Teil dieses Anspruchs ausgebildet ist.

Durch Aufstecken oder Aufkleben der Halterung wird eine einfache und schnelle Handhabung der Pinplatte erreicht. Da die Halterung auf der Oberseite vorgesehen ist, wird auch bei der Durchführung von Tests eine Behinderung weiterer Pinplatten, welche nebeneinander liegen, vermieden. Bei den Tests wird die Pinplatte an ihrer Halterung erfaßt und auf die Gegenplatte aufgelegt. Dadurch wird eine Berührung derjenigen Teile der Pinplatte, welche im Kontakt mit der Gegenplatte kommen, mit der Hand vermieden, so daß keine Verunreinigung insbesondere der Pinoberflächen auftritt und auch eine Kontamination des Anwenders vermieden wird. Zusätzlich wird eine gute mechanische Verbindung zwischen der Halterung und der Pinplatte durch das Aufstecken geschaffen. Bei Verwendung der Pinplatte kann die Halterung aufgesteckt werden und nach Durchführung der Tests gegebenenfalls wieder von der Pinplatte gelöst werden.

Die Halterung weist eine Grundplatte mit mindestens zwei Zapfen auf, wobei jeder Zapfen in eine zur Oberseite der Pinplatte hin offene Ausnehmung einsteckbar ist. Mit dieser Halterung kann insbesondere eine nur aus einer Pinreihe bestehende Pinplatte berührungsfrei transportiert werden, wobei die Halterung an der seitlichen Umrandung der Grundplatte angefaßt wird.

Es ist auch denkbar, die Halterung nur an der Oberseite der Pinplatte mittels Doppelklebestreifen aufzukleben und auf die Verwendung der Steckverbindung zu verzichten. Die Pinplatte ist dann mit einem Doppelklebestreifen an der Oberfläche versehen, so daß unterschiedliche Haltegriffe aufgeklebt werden können.

An der Oberseite der Grundplatte ist vorteilhafterweise ein Haltegriff angeordnet, um so die Handhabung zu vereinfachen.

Die Halterung mit oder ohne Haltegriff ist einstückig ausgebildet, wodurch die Handhabung weiter vereinfacht wird.

Werden bei den Tests größere Pinplatten verwendet, welche mehrere nebeneinanderliegende Pinreihen aufweisen, so sieht eine weitere Ausgestaltung der Erfindung vor, daß zwei zueinander parallele Reihen von Zapfen vorgesehen sind, wobei jede Reihe vier Zapfen aufweist. Durch die große Anzahl an Zapfen ist eine zuverlässige Verbindung gewährleistet, so daß sich die Halterung nicht versehentlich von der Oberfläche der Pinplatte lösen kann. Werden mit der Halterung mehrere nebeneinanderliegende Pinreihen erfaßt, so ermöglichen die parallelen Reihen von Zapfen außerdem einen gleichen parallelen Pinreihenabstand. Die Ausnehmungen, in welche die Zapfen eingreifen, können an allen denkbaren Stellen der Oberfläche der Pinplatten angeordnet sein. Die Pinplatte wird üblicherweise als Kunststofformteil ausgebildet, wobei die Oberseite recht dünn ist, so daß für eine gute Steckverbindung keine ausreichende Materialstärke vorliegt. Im Bereich der Pins ist jedoch sowohl eine ausreichende Materialstärke als auch eine ausreichende Länge für eine gute Steckverbindung vorhanden. Eine Weiterbildung der Erfindung sieht deshalb vor, daß in jedem Vorsprung eine zur Oberseite der Pinplatte hin offene Ausnehmung angeordnet ist. Die Ausnehmung kann sich über die gesamte Länge des Vorsprungs oder auch nur über eine Teillänge des Vorsprungs erstrecken. Zweckmäßigerweise ist jeder Zapfen der Form der Ausnehmung angepaßt, so daß eine paßgenaue Verbindung möglich ist.

Um die Halterung zusätzlich an der Oberfläche der Pinplatte zu sichern, sieht eine weitere Ausgestaltung der Erfindung vor, daß an der Unterseite der Grundplatte ein Doppelklebestreifen angeordnet ist. Dieser Doppelklebestreifen kann sowohl zwischen den Zapfen als auch seitlich der Zapfen angeordnet sein. Es können auch mehrere Doppelklebestreifen verwendet werden. Die Halterung kann auch nur aus einem Doppelklebeband an der Oberseite der Pinplatte bestehen. Das Klebeband auf der Pinplatte dient auch zur Stabilisierung von Schwächungslinien.

An dem Haltegriff ist zum Aufkleben an seiner Unterseite ein Doppelklebestreifen angeordnet.

Die Erfindung wird nachstehend anhand der Zeichnung näher erläutert. Die Zeichnung zeigt ein Ausführungsbeispiel der Erfindung. Hierbei stellen dar:

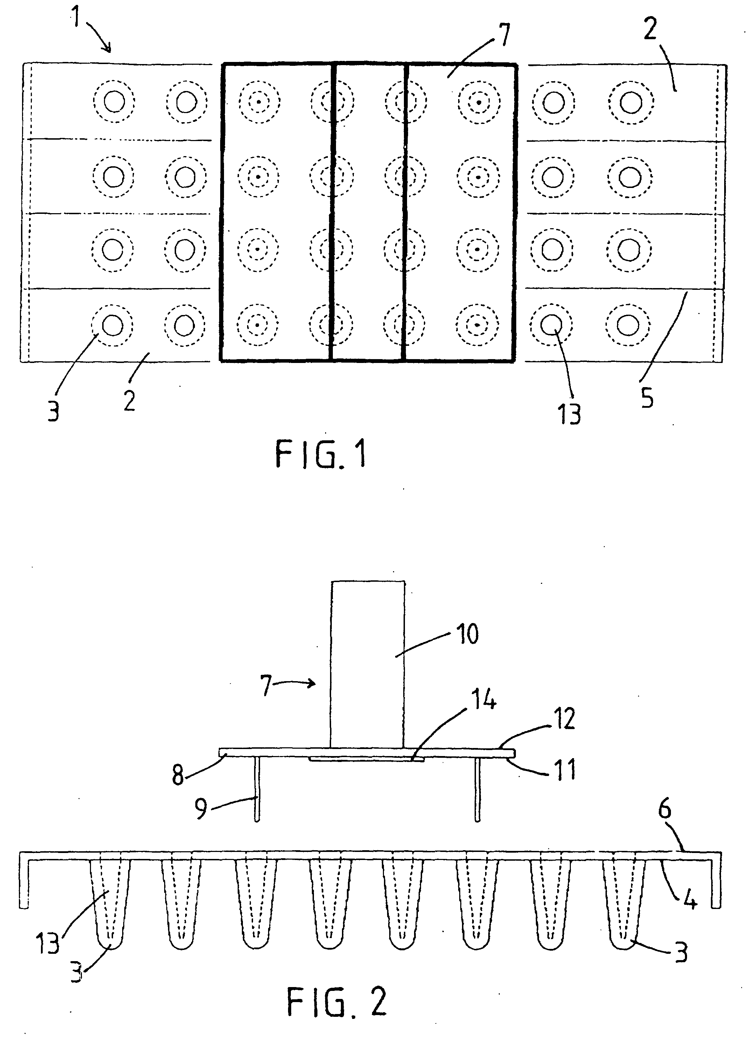

Fig. 1: eine Draufsicht auf die erfindungsgemäße Pinplatte,
Fig. 2: eine Seitenansicht nach Fig. 1 mit auseinandergezogener Halterung und Pinplatte,
Fig. 3: ein abgebrochener Querschnitt entlang der Linie I-I nach Fig. 1 und
Fig. 4: eine Seitenansicht einer Gegenplatte für die Pinplatte.

Die Pinplatte 1 weist vier nebeneinanderliegende Reihen 2 von Vorsprüngen 3 auf, welche auf einer Unterseite 4 der Pinplatte 1 angeordnet sind und sich nach unten erstrecken. Jede Reihe 2 ist von der angrenzenden Reihe 2 durch eine Schwächungslinie 5 getrennt, welche ein Teilen der Pinplatte 1 entlang dieser Schwächungslinie 5 ermöglicht. Auf der Oberseite 6 der Pinplatte 1 ist eine Halterung 7 angeordnet. Die Halterung 7 weist eine Grundplatte 8, Zapfen 9 und einen Haltegriff 10 auf. Die Zapfen 9 sind in zwei parallelen Reihen angeordnet. Jede Reihe weist vier Zapfen 9 auf, welche an der Unterseite 11 der Grundplatte 8 angeordnet sind. Auf der Oberseite 12 der Grundplatte 8 ist der Haltegriff 10 vorgesehen. Die Grundplatte 8, die Zapfen 9 und der Haltegriff 10 sind als Kunststofformteil einstückig ausgebildet, wobei die Zapfen 9 eine gleiche Form haben. Ebenso sind die Vorsprünge 3 identisch ausgebildet.

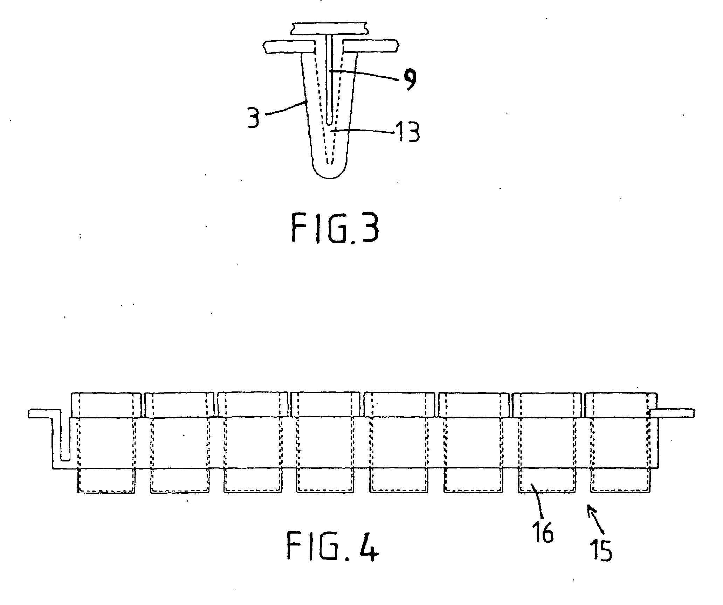

Jeder Vorsprung 3 hat eine sich nach unten verjüngende konische Form und weist eine zur Oberseite 6 der Pinplatte 1 hin offene Ausnehmung 13 auf. Die Ausnehmung 13 verläuft ebenfalls konisch und verjüngt sich zur Spitze des Vorsprungs 3 hin. Die Halterung 7 ist auf die Pinplatte 1 aufgesteckt, wobei jeder Zapfen 9 in eine Ausnehmung 13 eines Vorsprungs 3 hineinragt und sich innerhalb der Ausnehmung 13 verklemmt. Wie aus Figur 3 ersichtlich, hat der Zapfen 9 eine stiftartige Ausbildung mit konstantem Querschnitt. Durch die konische Ausbildung der Ausnehmung 13 wird ein Verklemmen des Zapfens 9 ermöglicht. Der Zapfen 9 kann jedoch auch konisch ausgebildet sein und der Form der Ausnehmung 13 angepaßt sein, so daß eine paßgenaue Verbindung zwischen dem Zapfen 9 und der Ausnehmung 13 ermöglicht wird. Als zusätzliche Sicherung ist auf der Unterseite 11 der Grundplatte 8 ein Doppelklebestreifen 14 zwischen den beiden Reihen der Zapfen 9 angebracht. Ist die Halterung 7 auf der Pinplatte 1 aufgesteckt, so klebt der Doppelklebestreifen 14 auf der Oberseite 6 der Pinplatte 1 und gewährleistet eine zusätzliche Klebeverbindung.

Bei der Durchführung von Tests wie beispielsweise Enzymimmunoassays ist die Oberfläche der Vorsprünge 3 mit immobilisierten Testreagenzien beschichtet. Beim Test wird die Pinplatte 1 auf eine in Figur 4 dargestellte Gegenplatte 15 aufgesetzt. Die Gegenplatte 15 weist in regelmäßigen Reihen angeordnete Näpfchen 16 auf. In den Näpfchen 16 befindet sich die zu untersuchende Flüssigkeit. Die Näpfchen 16 sind alle gleich tief ausgebildet und beim Aufsetzen der Pinplatte 1 auf die Gegenplatte 15 taucht jeder Vorsprung 3 in ein Näpfchen 16 ein, wodurch eine Reaktion in der zu untersuchenden Flüssigkeit ausgelöst wird. Damit die Vorsprünge 3 nicht verunreinigt werden, wird die Pinplatte 1 mittels der aufgesteckten Halterung 7 transportiert. Die Pinplatte 1 kann somit problemlos auf die Gegenplatte 16 aufgelegt und von dieser wieder entfernt werden. Außerdem wird auch eine Kontamination der Finger des Anwenders beim Waschen der Pinplatte vermieden, da die Pinplatte an ihrer Oberseite mittels der Halterung erfaßt wird.

## Patentansprüche

1. Pinplatte zur Durchführung von Tests in Flüssigkeiten mit in mindestens einer Reihe angeordneten Vorsprüngen (3) auf der Unterseite (4), wobei eine Halterung (7) zum Transport der Pinplatte (1) vorgesehen ist,
**dadurch gekennzeichnet, daß** die Halterung (7) auf die Oberseite (6) der Pinplatte (1) aufsteckbar ist, wobei die Halterung (7) eine Grundplatte (8) mit mindestens zwei Zapfen (9) aufweist, wobei jeder Zapfen (9) in eine zur Oberseite (6) der Pinplatte (1) hin offene Ausnehmung (13) einsteckbar ist, oder daß die Halterung (7) auf die Oberseite (6) der Pinplatte (1) aufklebbar ist, wobei ein Doppelklebestreifen (14) an der Oberseite (6) der Pinplatte (1) vorgesehen ist.

2. Pinplatte nach Anspruch 1,
**dadurch gekennzeichnet,**
**daß** an der Oberseite (12) der Grundplatte (8) ein Haltegriff (10) angeordnet ist.

3. Pinplatte nach einem der Ansprüche 1 und 2,
**dadurch gekennzeichnet,**
**daß** zwei zueinander parallele Reihen von Zapfen (9) vorgesehen sind, wobei jede Reihe vier Zapfen (9) aufweist.

4. Pinplatte nach einem der vorhergehenden Ansprüche,
**dadurch gekennzeichnet,**
**daß** in jedem Vorsprung (3) eine zur Oberseite (6) der Pinplatte (1) hin offene Ausnehmung (13) angeordnet ist.

5. Pinplatte nach einem der vorhergehenden Ansprüche,
**dadurch gekennzeichnet,**
**daß** jeder Zapfen (9) der Form der Ausnehmung (13) angepaßt ist.

6. Pinplatte nach einem der vorhergehenden Ansprüche
**dadurch gekennzeichnet,**
**daß** an der Unterseite (11) der Grundplatte (8) ein Doppelklebestreifen (14) angeordnet ist.

7. Pinplatte nach einem der vorhergehenden Ansprüche,
**dadurch gekennzeichnet,**
**daß** die Halterung (7) aus einem Haltegriff (10) besteht.

8. Pinplatte nach Anspruch 7,
**dadurch gekennzeichnet,**
**daß** an der Unterseite des Hattegriffs (10) ein Doppelklebestreifen (14) angeordnet ist.

## Claims

1. Pin plate for the performance of tests in fluids, with projections (3) arranged in at least one row on the underside (4), a holder (7) being provided to transport the pin plate (1), **characterised in that** the holder (7) can be placed on the top (6) of the pin plate (1), the holder (7) having a base plate (8) with at least two pegs (9), wherein each peg (9) can be inserted in a recess (13) which is open towards the top (6) of the pin plate (1), or **in that** the holder (7) can be glued to the top (6) of the pin plate (1), a double adhesive strip (14) being provided on the top (6) of the pin plate (1).

2. Pin plate according to claim 1, **characterised in that** a handle (10) is arranged on the top (12) of the base plate (8).

3. Pin plate according to one of claims 1 and 2, **characterised in that** two parallel row of pegs (9) are provided, each row having four pegs (9).

4. Pin plate according to any of the previous claims, **characterised in that** arranged in each projection (3) is a recess (13) which is open towards the top (6) of the pin plate (1).

5. Pin plate according to any of the previous claims, **characterised in that** each peg (9) is adapted to the form of the recess (13).

6. Pin plate according to any of the previous claims, **characterised in that** a double adhesive strip (14) is arranged on the underside (11) of the base plate (8).

7. Pin plate according to any of the previous claims, **characterised in that** the holder (7) comprises a handle (10).

8. Pin plate according to claim 7, **characterised in that** a double adhesive strip (14) is arranged on the underside of the handle (10).

## Revendications

1. Plaque à pointes pour exécuter des tests dans des liquides, comportant des protubérances (3) agencées en au moins une rangée sur la face inférieure (4), sachant qu'un support (7) est prévu pour le transport de ladite plaque à pointes (1),
**caractérisée par le fait que** le support (7) peut être emboîté sur la face supérieure (6) de la plaque à pointes (1), ledit support (7) présentant une plaque d'embase (8) munie d'au moins deux broches (9), chaque broche (9) pouvant être enfichée dans un évidement (13) ouvert vers la face supérieure (6) de la plaque à pointes (1) ; ou **par le fait que** le support (7) peut être collé sur la face supérieure (6) de la plaque à pointes (1), une bande adhésive double (14) étant prévue à la face supérieure (6) de ladite plaque à pointes (1).

2. Plaque à pointes selon la revendication 1,
**caractérisée par le fait**
**qu'**une poignée de maintien (10) est disposée à la face supérieure (12) de la plaque d'embase (8).

3. Plaque à pointes selon l'une des revendications 1 et 2,
**caractérisée**
**par** la présence de deux rangées de broches (9) parallèles l'une à l'autre, chaque rangée comptant quatre broches (9).

4. Plaque à pointes selon l'une des revendications précédentes,
**caractérisée par le fait**
**qu'**un évidement (13), ouvert vers la face supérieure (6) de ladite plaque à pointes (1), est pratiqué dans chaque protubérance (3).

5. Plaque à pointes selon l'une des revendications précédentes,
**caractérisée par le fait**
**que** chaque broche (9) est adaptée à la forme de l'évidement (13).

6. Plaque à pointes selon l'une des revendications précédentes,
**caractérisée par le fait**
**qu'**une bande adhésive double (14) est disposée à la face inférieure (11) de la plaque d'embase (8).

7. Plaque à pointes selon l'une des revendications précédentes,
**caractérisée par le fait**
**que** le support (7) est constitué d'une poignée de maintien (10).

8. Plaque à pointes selon la revendication 7,
**caractérisée par le fait**
**qu'**une bande adhésive double (14) est disposée à la face inférieure de la poignée de maintien (10).
